# EUROPEAN PATENT SPECIFICATION

(11) **EP 1 576 976 B1**
(45) Date of publication and mention of the grant of the patent: **10.12.2008**
(21) Application number: 05005379.2
(22) Date of filing: 11.03.2005
(51) Int. Cl.: A61M 5/152

(54) **Patient-controlled analgesia (PCA) apparatus capable of continuous injection and additional administration of drugs solution**
Patienten-kontrollierte Analgesievorrichtung für die kontinuierliche Injektion und Abgabe zusätzlicher Medikamentenlösung
Analgésie régulée par le patient pour la perfusion continuée et l'administration additionnelle d'une solution médicamenteuse

(30) Priority: 12.03.2004 JP 2004071102
(43) Date of publication of application: 21.09.2005
(73) Proprietor: Nipro Corporation, Kita-ku, Osaka-shi, Osaka-fu, 531-8510 (JP)
(72) Inventor: Hiejima, Katsuhiro, Osaka-shi Osaka-fu, 531-8510 (JP); Ebara, Yukinori, Osaka-shi Osaka-fu, 531-8510 (JP); Mori, Takeshi, Osaka-shi Osaka-fu, 531-8510 (JP); Hino, Satoki, Osaka-shi Osaka-fu, 531-8510 (JP); Yanagisono, Yoshinori, Osaka-shi Osaka-fu, 531-8510 (JP)
(74) Representative: Banzer, Hans-Jörg

(56) References cited:
- EP-A- 0 783 895
- WO-A-02/098493
- US-B1- 6 206 850

## Description

### BACKGROUND OF THE INVENTION

The present invention relates to a patient-controlled analgesia (PCA) apparatus capable of continuous injection and additional administration of a drug solution and, more specifically, to a PCA apparatus which is capable of additional administration simultaneously with continuous injection, in which a drug solution can be administered additionally via a release valve while being injected continuously in a slight amount via a flow rate control tube, the period of drug solution injection can be controlled by changing the length of the flow rate control tube, the amount of administration can be selected as desired by changing the size of a sub-reservoir, the line is simple, operation is easy, defects such as leakage are prevented, and manufacturing cost is low.

### BACKGROUND ART

In recent years, in the field of anesthesiology, an epidural catheter insertion method using an instrument which can inject drug solution continuously in a slight amount is used extensively in order to alleviate postoperative pain or cancer pain. In such an approach, symptoms of patients vary on an individual basis, and the patient may experience sudden pain even while a predetermined amount of pain reliever is being injected continuously in a slight amount. In order to cope with such an emergency case, development of devices by which the patient can administer the pain reliever in one shot to himself/herself to mitigate the pain is now in progress. As one of such devices, a drug solution self-injection tool (JP-T-63-501195) or a mobile pain killing device (WO95/27521), provided with a control device which is operated by a patient in order to administer a drug solution selectively from a dose reservoir is known.

In the drug solution self-injection tool (JP-T-63-501195), since a flexible sheet can hardly be restored to its original state of the tool even when a patient releases his/her finger from a push-button, refilling of drug solution into a chamber is performed only by a force to discharge drug solution from a drug solution container (the container is adapted to discharge drug solution encapsulated in a balloon by a restoring force of the balloon). However, since the drug solution container is intended to be used for administering a drug solution in a slight amount, the amount of discharge therefrom is very small. Therefore, it takes for a long time for refilling of the drug solution. In other words, the drug solution self-injection tool in the related art has a disadvantage in that it takes time for refilling, and hence subsequent injection of drug solution cannot be done immediately. In addition to the above-described disadvantage of taking long to refill, there is another disadvantage in that the applied dose is limited, and hence the applied dose cannot be selected as desired, whereby the drug solution cannot be injected in the amount required by the patient. Also, the drug solution self-injection tool in the related art is complex in structure, and hence costs much to manufacture, and has fundamental defects such that leakage tends to occur at the periphery of the flexible sheet.

The mobile pain killing device (WO95/27521) is a device in which a drug solution continuous injector, an opening-closing valve, a three-way connector, and a pressure one-way valve are connected in line, and a reservoir is connected vertically to the line in series via the three-way connector, and in which the drug solution continuous injector having a predetermined capacity or duration in advance is prepared, and the drug solution is injected by a predetermined dose depending on the symptom of the patient. Subsequently, the opening-closing valve is opened for transferring the drug solution into the reservoir for a predetermined period by a flow rate control device. When the patient feels pain, the patient presses the reservoir by himself/herself and injects the drug solution in the reservoir into the body via the pressure one-way valve. Since the mobile pain killing device is provided with the opening-closing valve in line as described above, drug solution flown out from the drug solution continuous injector can be cut off easily by the patient, and thus there is a risk that the patient cannot administer the drug solution in an accurate amount. Furthermore, although it is also applied to the above-described drug solution self-injection tool, these devices are intended for bolus administration of a drug solution to the patient, and hence they have a disadvantage in that they cannot administer the drug solution continuously to the patient.
Documents WO 02/098493 A, US 6,206,850 B1 and EP 0 783 895 A1 each disclose a patient-controlled analgesia apparatus capable of simultaneous continuous injection and additional administration of a drug solution. Each of the devices disclosed in these documents comprise one release valve which is openable at a certain pressure. Moreover, WO 02/098493 discloses an apparatus according to the preamble of claim 1.

In view of such circumstances, it is an object of the invention to provide a PCA apparatus capable of continuous injection and additional administration of a drug solution and, more specifically, to a PCA apparatus which is capable of additional administration simultaneously with continuous injection, in which a drug solution can be additionally administrated via a release valve while being injected continuously in slight amount via a flow rate control tube, the period of filling of the drug solution can be controlled by changing the length of the flow rate control tube, a dose can be selected as desired by changing the size of a sub-reservoir, the line is simple, operation is easy, defects such as leakage are prevented, and manufacturing cost is low.

In order to solve the above-described problem, the inventors, after having devoted themselves to study, invented a PCA apparatus including a flow rate control device having a release valve to be opened by a certain pressure for allowing drug solution to be flown out, and a flow rate control tube for controlling the flow rate of drug solution flowing out from a reservoir by resistance in a duct line, the flow rate control device being provided not only in a drug solution distribution tube for continuous injection, but also in a drug solution distribution tube for additional administration, wherein additional administration can be conducted during continuous injection of a drug solution, a period of filling a drug solution to a sub-reservoir can be controlled by changing the length of the flow rate control tube, the amount of additional administration can be selected as desired by changing the size of the sub-reservoir, the line is simple, operation is easy, defects such as leakage are prevented, and manufacturing cost is low. In the present invention, it is preferable to provide a check valve for preventing drug solution discharged from the sub-reservoir by the patient from flowing in the reverse direction at the time of additional administration.

### SUMMARY OF THE INVENITON

The above object is achieved by a patient-controlled analgesia (PCA) apparatus as defined by independent claim 1. The dependent claim defines a preferred embodimentments of the invention. In other words, the present invention is a patient-controlled analgesia (PCA) apparatus capable of continuous injection and additional administration of a drug solution including (i) a reservoir for storing the drug solution in a pressurized state and allowing the drug solution to flow in and out to/from a drug solution port, (ii) a drug solution distribution tube for continuous injection extending from the drug solution port, (iii) a flow rate control device A including a release valve disposed in the drug solution distribution tube for continuous injection and which is openable at a certain pressure for allowing the drug solution to flow out and a flow rate control tube for adjusting a flow rate of drug solution flowing out from the reservoir by resistance in a duct line, (iv) a drug solution distribution tube for additional administration branched from the drug solution distribution tube between the reservoir and the flow rate control device A, (v) a flow rate control device B including a release valve disposed in the drug solution distribution tube for additional administration and which is openable by a certain pressure for allowing the drug solution to flow out and a flow rate control tube for adjusting the flow rate of the drug solution flowing out from the reservoir by resistance in the duct line, and (vi) a sub-reservoir capable of being pressed and deformed provided at the distal end of the drug solution distribution tube for additional administration.

In the present invention, it is also possible to provide a check valve for preventing the drug solution from flowing in the reverse direction to the reservoir at the time of additional administration provided in the drug solution distribution tube for continuous injection between the drug solution distribution tube for additional administration and the reservoir.

In the present invention, the drug solution tube for continuous injection extending from the drug solution port includes a portion to be connected to the drug solution port of the reservoir and a portion to be connected to a flow rate control device, and is connected via a three-way connector to the drug solution distribution tube for additional administration.

In the present invention, a drug solution discharged by pressure of a balloon, which is commonly used as a reservoir, flows through the drug solution distribution tube for continuous injection, passes through the flow rate control tube in the flow rate control device provided in the drug solution distribution tube for continuous injection and is injected into the patient continuously in a slight amount, and simultaneously, the drug solution also flows through the drug solution distribution tube for additional administration, passes through the flow rate control tube in the flow rate control device provided in the drug solution distribution tube for additional administration, and is filled in the sub-reservoir. Then, drug solution discharged from the sub-reservoir by being pressed and deformed by a force of the patient's hand at the time of additional administration passes through the release valve in the flow rate control device provided in the drug solution distribution tube for additional administration, is prevented by the check valve from flowing in the reverse direction to the balloon, passes the release valve in the flow rate control device provided in the drug solution distribution tube for continuous injection, and is additionally administered to the patient, that is, additional administration can be conducted while continuously injecting drug solution by the apparatus characterized by the flow rate control device. Also, by employing the PCA apparatus according to the present invention, in addition to the capability of conducting additional administration during continuous injection of a drug solution, the period of filling the sub-reservoir with the drug solution can be controlled by changing the length of the flow rate control tube, the amount of additional administration can be selected as desired by changing the size of the sub-reservoir, the line is simplified, operation is easy, and defects such as leakage can be prevented. Consequently, it is suitable for additional administration for a drug solution and, in addition, since the manufacturing cost is low, the economic burden of the patient can be alleviated.

### BRIEF DESCRIPTION OF THE DRAWINGS

Fig. 1 is a plan view of an embodiment of a PCA apparatus capable of continuous injection and additional administration of drug solution of the present invention.

Fig. 2 is a vertical cross-sectional view of an embodiment of a flow rate control device provided in the drug solution distribution tube for continuous injection and additional administration of the PCA apparatus capable of continuous injection and additional administration of drug solution shown in Fig. 1.

Fig. 3 is a vertical cross-sectional view of another embodiment of a flow rate control device provided in the drug solution distribution tube for continuous injection and additional administration of the PCA apparatus capable of continuous injection and additional administration of drug solution shown in Fig. 1.

Fig. 4 is a plan view of an embodiment of the PCA apparatus capable of continuous injection and additional administration using the flow rate control device shown in Fig. 2.

Fig. 5 is a vertical cross-sectional view of flow rate control devices 4 and 6 provided in the drug solution distribution tube for continuous injection and additional administration shown in Fig. 4 illustrating the state in which continuous injection is being conducted.

Fig. 6 is a vertical cross-sectional view of the flow rate control devices 4 and 6 provided in the drug solution distribution tube for continuous injection and additional administration shown in Fig. 4, illustrating the state in which continuous injection and additional administration are simultaneously being conducted.

Fig. 7 is a plan view of an embodiment of the PCA apparatus capable of continuous injection and additional administration of drug solution using the flow rate control device shown in Fig. 3.

Fig. 8 is a vertical cross-sectional view of the flow rate control devices 4 and 6 provided in the drug solution distribution tube for continuous injection and additional administration shown in Fig. 7 illustrating the state in which the continuous injection is being conducted.

Fig. 9 is a vertical cross-sectional view of the flow rate control devices 4 and 6 provided in the drug solution distribution tube for continuous injection and additional administration shown in Fig. 7 illustrating the state in which continuous injection and additional administration are simultaneously being conducted.

### DETAILED DESCRIPTION OF THE INVENTION

A PCA apparatus according to the present invention is suitable as an apparatus to be connected to a drug solution continuous injector with a balloon (a device being used as means for administering a drug solution, such as a pain reliever, an anesthetic drug, an antibiotic, or an anticancer drug, continuously in a slight amount to blood vessels, a subcutaneous portion or an epidural portion, wherein the drug solution is filled into a balloon formed of a rubbery resilient material and the drug solution is injected by utilizing a contracting force of the balloon), for conducting additional administration of a certain amount of the drug solution depending on the pain of the patient during continuous injection of a certain amount of the drug solution.

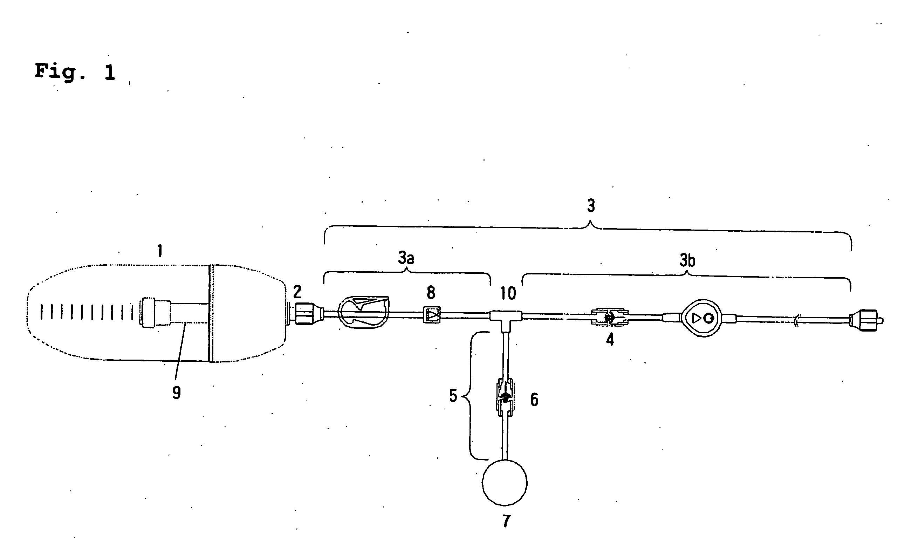

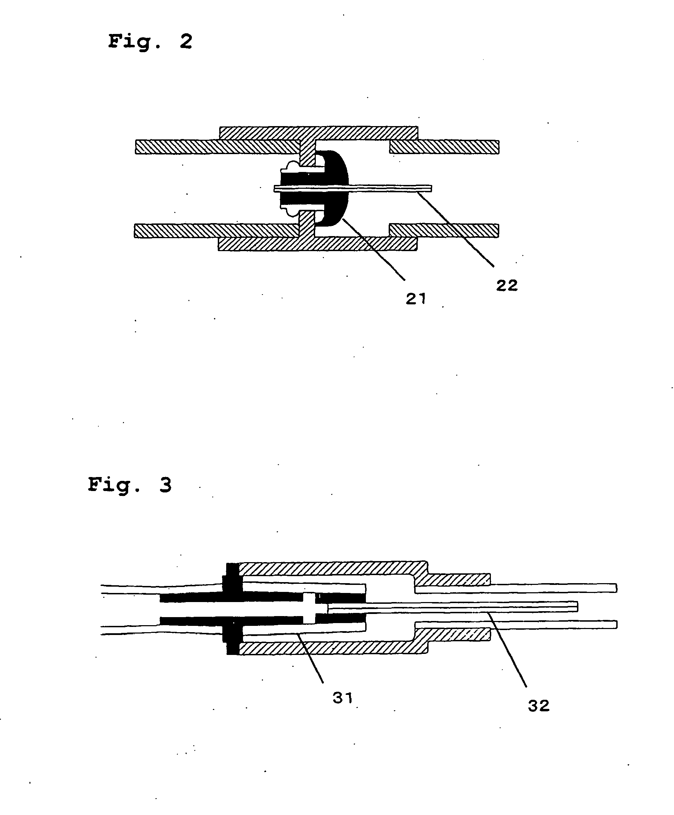

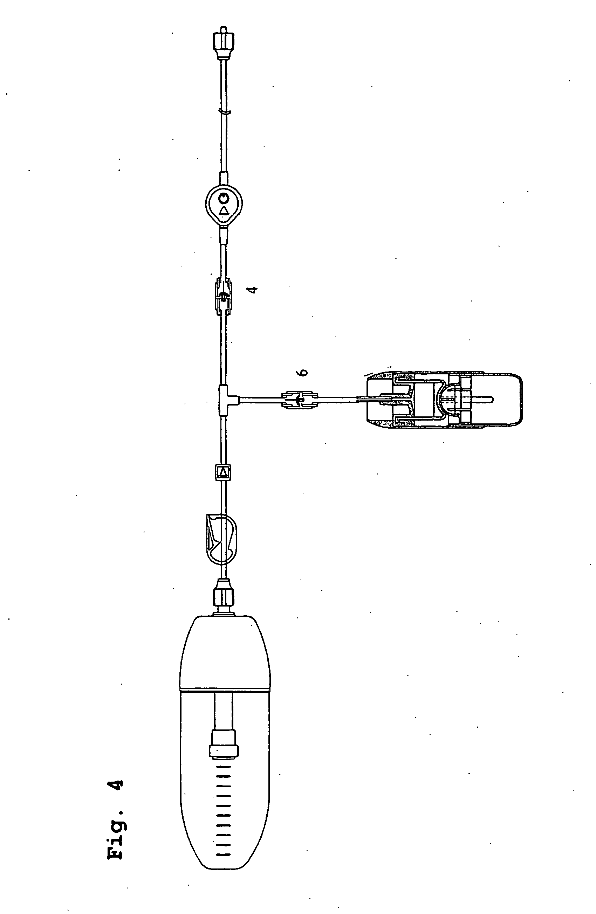

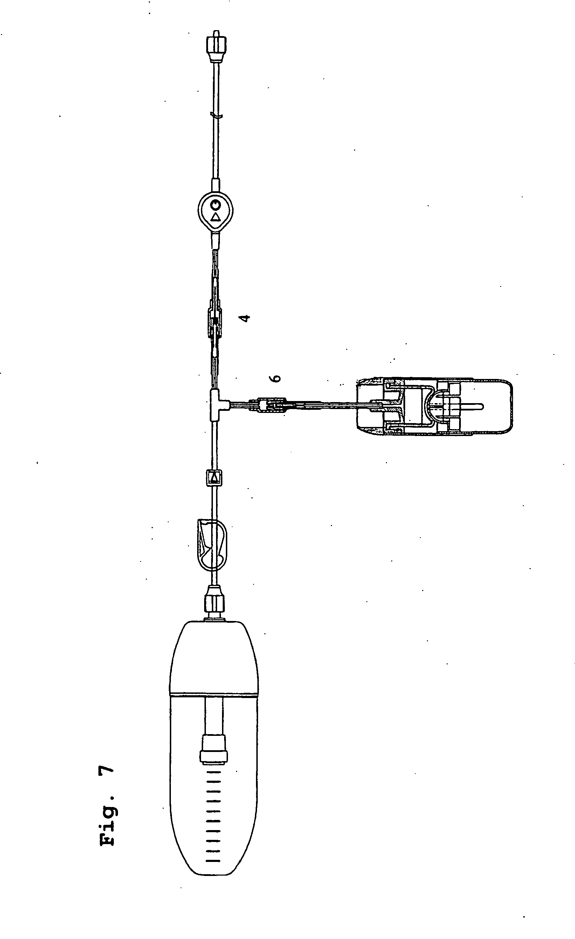

Subsequently, referring to the drawings, an embodiment of the present invention will be described. Fig. 1 is a schematic drawing showing a PCA apparatus according to the present invention, which is capable of continuous injection and additional administration of drug solution, shown in a state of being connected to a reservoir, Fig. 2 is a vertical cross-sectional view showing an embodiment of the flow rate control devices 4 and 6 of the PCA apparatus as shown in Fig.1, and likewise, Fig. 3 is a vertical cross-sectional view showing another embodiment of the flow rate control devices 4 and 6 shown in Fig. 1. Fig. 4 is a PCA apparatus according to the present invention using the flow rate control devices shown in Fig. 2, and Fig. 5 and Fig. 6 are vertical cross-sectional views for explaining the function of the flow rate control devices 4 and 6 in the embodiment of the PCA apparatus of Fig. 4 in more detail. Furthermore, Fig. 7 is a PCA apparatus according to the present invention using the flow rate control device shown in Fig. 3, and Fig. 8 and Fig. 9 are vertical cross-sectional views for explaining the function of the flow rate control devices 4 and 6 in the embodiment of the PCA apparatus of Fig. 7 in more detail.

As shown in Fig. 1, the PCA apparatus of the present invention includes a reservoir 1 for storing a drug solution in a pressurized state and allowing the drug solution to flow in and out from a drug solution port 2, a drug solution distribution tube 3 for continuous injection extending from the drug solution port 2, a flow rate control device 4 including a release valve disposed in the drug solution distribution tube 3 for continuous injection which is opened by a certain pressure and allows drug solution to flow out and a flow rate control tube for adjusting the flow rate of the drug solution flowing out from the reservoir 1 by resistance in a duct line, a drug solution distribution tube 5 for additional administration branched from the drug solution distribution tube 3 between the reservoir 1 and the flow rate control device 4, the flow rate control device 6 as described above disposed in the drug solution distribution tube 5 for additional administration, and a sub-reservoir 7 capable of being pressed and deformed provided at the distal end of the drug solution distribution tube 5 for additional administration, and further a check valve 8 for preventing drug solution from flowing in the reverse direction toward the reservoir 1 during additional administration and which is provided between the drug solution distribution tube 5 for additional administration and the reservoir 1.

Fig. 2 is a vertical cross-sectional view of the flow rate control device 4 or 6, which is a characteristic part of the present invention, according to the embodiment, including an umbrella valve 21 which is opened by a certain pressure and allows drug solution to flow out, and a flow rate control tube 22 for adjusting the flow rate of the drug solution flowing out from the reservoir by resistance in the duct line.

Fig. 3 is a vertical cross-sectional view of the flow rate control device 4 or 6, which is a characteristic part of the present invention, according to another embodiment, including a rubbery resilient film 31 which is opened by a certain pressure and allows drug solution to flow out, and a flow rate control tube 32 for adjusting the flow rate of the drug solution flowing out from the reservoir by resistance in the duct line.

The flow rate control device 4 or 6 either includes the release valve 21 which is opened by a certain pressure and allows drug solution to flow out and the flow rate control tube 22 for adjusting the flow rate of drug solution flowing out from the reservoir 1 by resistance in the duct line, or includes the release valve 31 which is opened by a certain pressure and allows drug solution to flow out and the flow rate control tube 32 for adjusting the flow rate of the drug solution flowing out from the reservoir 1 by resistance in the duct line. The flow rate control device 4 or 6 may have the same release valve and flow rate control tube, or may have a release valve and a flow rate control tube different from each other.

Drug solution discharged by the pressure of a balloon 9 in the reservoir 1 flows in the drug solution distribution tube 3 for continuous injection, passes through the flow rate control tube 22 or 32 in the flow rate control device 4 provided in the drug solution distribution tube 3 for continuous injection, and is injected into a patient continuously in a slight amount, and simultaneously, the drug solution also flows through the drug solution distribution tube 5 for additional administration, passes through the flow rate control tube 22 or 32 in the flow rate control device 6 for additional administration, and is filled in the sub-reservoir 7. Then, the drug solution discharged by the force of the patient's hand during additional administration passes through the release valve 21 or 31 in the flow rate control device 6 provided in the drug solution distribution tube 5 for additional administration, is prevented by the check valve 8 from flowing in the reverse direction to the balloon 9, passes through the release valve 21 or 31 in the flow rate control device 4 provided in the drug solution distribution tube 3 for continuous injection and is additionally administered to the patient.

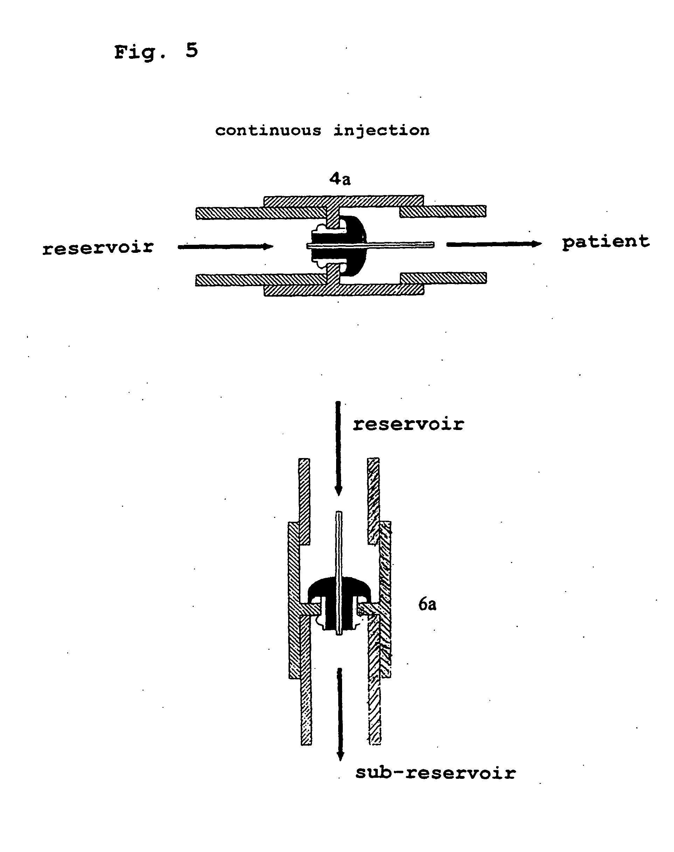

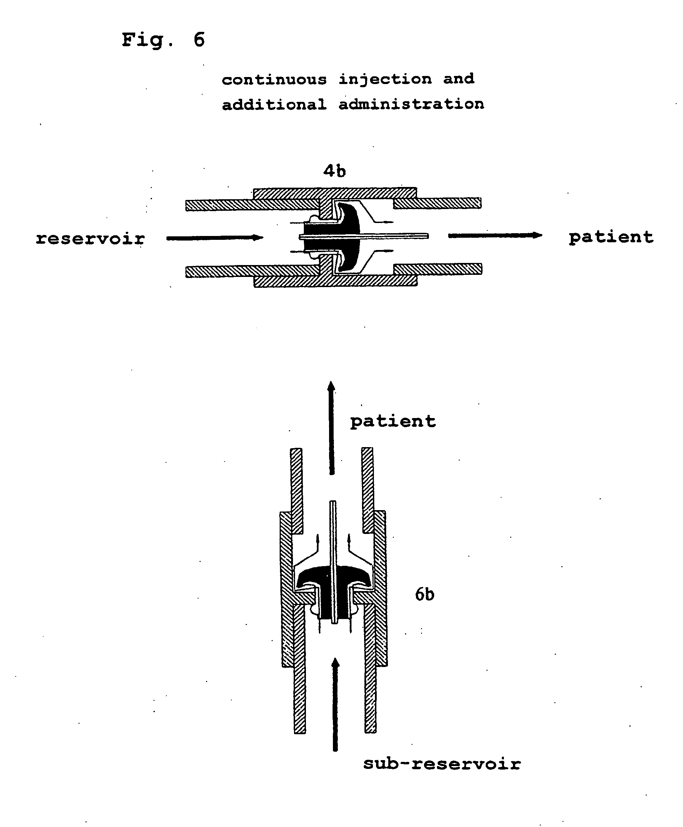

Fig. 4 shows an embodiment in which the flow rate control device shown in Fig. 2 is used in the flow rate control devices 4 and 6 in Fig. 1, and Fig. 5 and Fig. 6 are vertical cross-sections for explaining the function of the flow rate control devices 4 and 6 in the PCA apparatus shown in Fig. 4 more in detail. Fig. 5 shows a flow (4a) of drug solution in the flow rate control device 4 and a flow (6a) of drug solution in the flow rate control device 6 during continuous injection. Fig. 6 shows a flow (4b) of drug solution in the flow rate control device 4 and a flow (6b) of drug solution in the flow rate control device 6 during simultaneous continuous injection and additional administration.

In the flow rate control devices 4 and 6 during continuous injection, drug solution discharged by the pressure of the balloon 9 in the reservoir 1 passes through the flow rate control tube 22 in the flow rate control device 4 provided in the drug solution distribution tube 3 for continuous injection in the direction indicated by arrows, and is administered continuously to the patient in a slight amount and, simultaneously, passes through the flow rate control tube 22 in the flow rate control device 6 provided in the drug solution distribution tube 5 for additional administration in the direction indicated by arrows, and is stored in the sub-reservoir 7. When the patient presses the sub-reservoir 7 depending on the degree of pain, that is, when the drug solution is administered additionally while being injected continuously, the sub-reservoir 7 is deformed, and the drug solution is discharged from the sub-reservoir 7, passes through the flow rate control tube 22 and the umbrella valve 21 in the flow rate control device 6 provided in the drug solution distribution tube 3 for additional administration in the direction indicated by arrows, and passes through the flow rate control tube 22 and the umbrella valve 21 in the flow rate control device 4 provided in the drug solution distribution tube 3 for continuous administration, whereby the drug solution flows to the patient.

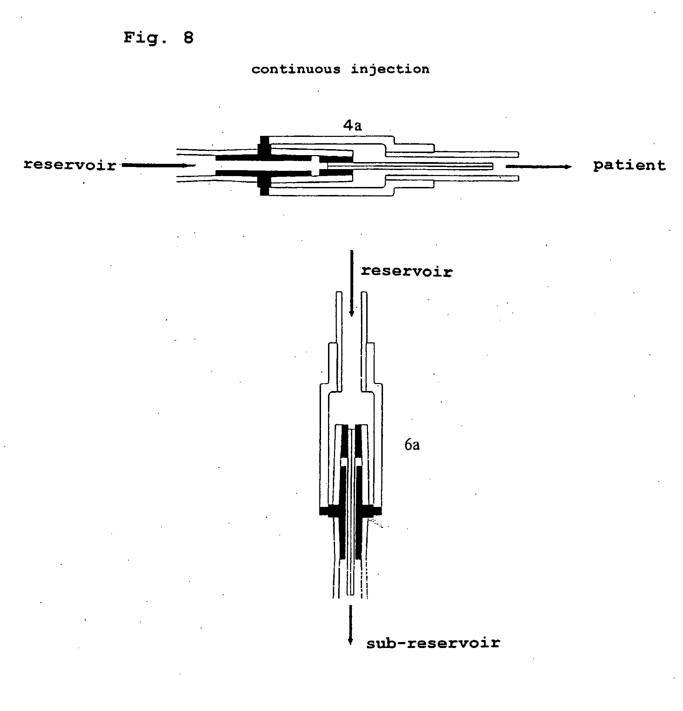

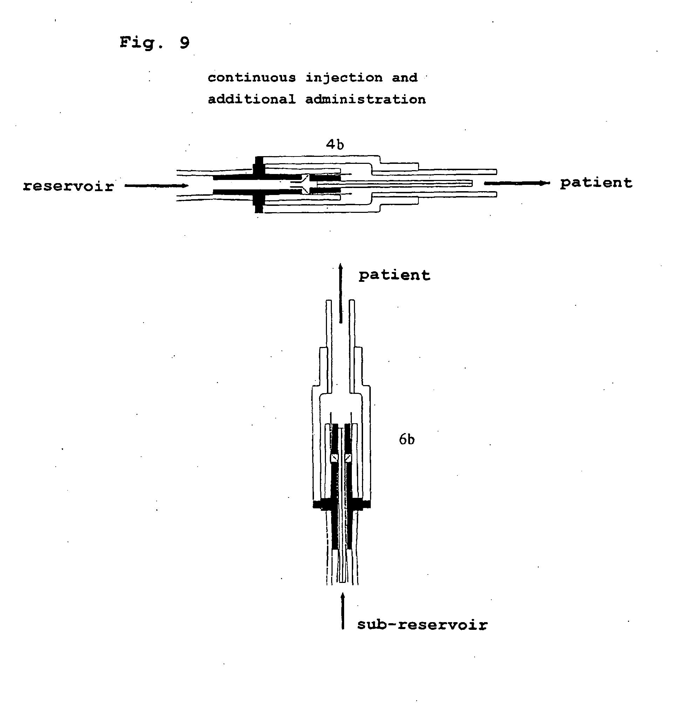

Fig. 7 shows an embodiment in which the flow rate control device shown in Fig. 3 is used for the flow rate control devices 4 and 6 in Fig. 1, and Fig. 8 and Fig. 9 are vertical cross-sectional views for explaining the function of the flow rate control devices 4 and 6 shown in the PCA apparatus shown in Fig. 7 in more detail. Fig. 8 shows a flow (4a) of drug solution in the flow rate control device 4 and a flow (6a) of drug solution in the flow rate control device 6 during continuous injection. Fig. 9 shows a flow (4b) of drug solution in the flow rate control device 4 and a flow (6b) of drug solution in the flow rate control device 6 during the simultaneous continuous injection and additional administration.

In the flow rate control devices 4 and 6 during continuous injection, drug solution discharged by the pressure of the balloon 9 in the reservoir 1 passes through the flow rate control tube 32 in the flow rate control device 4 provided in the drug solution distribution tube 3 for continuous injection in the direction indicated by arrows, and is administered continuously to the patient in a slight amount and, simultaneously, passes through the flow rate control tube 32 in the flow rate control device 6 provided in the drug solution distribution tube 5 for additional administration, and is stored in the sub-reservoir 7. When the patient presses the sub-reservoir 7 depending on the degree of pain, that is, when the drug solution is additionally administered during continuous injection, the sub-reservoir 7 is pressed and deformed, and hence the drug solution is forced out of the sub-reservoir 7, passes through the flow rate control tube 32 and the rubbery resilient film 31 in the flow rate control device 6 provided in the drug solution distribution tube 3 for additional administration in the direction indicated by arrows, and passes through the flow rate control tube 32 and the rubbery resilient film 31 in the flow rate control device 4 provided in the drug solution distribution tube 3 for continuous injection, whereby the drug solution flows to the patient.

The respective components will be described in further detail below.

The reservoir 1 used in the present invention for storing drug solution in a pressurized state and allowing the drug solution to flow in and out from the drug solution port 2 is a chamber in which the drug solution is stored, and includes a pressurizing mechanism for discharging or forcing the stored drug solution toward the outside. An outer wall of the chamber in which drug,solution is stored is formed with at least one opening for communicating between the inside and the outside of the chamber. Drug solution is filled through the opening from the outside, and the stored drug solution is discharged toward the outside. A known drug solution reservoir is disclosed in U.S. Patent No. 4386929, and specifically, a balloon formed of a resilient body which is adapted to discharge drug solution stored therein by its own contracting force is preferably used. The material used for the balloon is not specifically limited as long as it has high elasticity, and hence can provide an internal pressure required for discharging liquid, and can be used for medical applications. However, it is preferable to employ rubber, synthetic rubber, or an elastomer. The inner wall of the balloon is preferably covered by a chemically resistant material so as not to be changed in its nature by the filled drug solution. The reservoir is not limited thereto.

To store the drug solution in a pressurized state means to store the drug solution in the balloon 9 in the reservoir 1, and the drug solution in the pressurized state is discharged toward the patient and produces a flow of the drug solution. The drug solution in the pressurized state is preferably set to be pressurized to the order of 50 to 100 KPa and discharged toward the patient.

In order to allow drug solution to flow in and out from the drug solution port 2, the drug solution is filled into the balloon 9 in the reservoir 1 via the drug solution port 2 by a syringe or the like. The drug solution stored in the balloon 9 is enabled to flow toward the patient by connecting a specific connector disposed on the other end of the drug solution distribution tube 3 for continuous injection with the drug solution port 2 of the reservoir 1.

The drug solution distribution tube 3 for continuous injection and the drug solution distribution tube 5 for additional administration used in the present invention are suitable for distributing drug solution discharged by the balloon 9 in the reservoir 1 to the patient, and is formed of soft polyvinyl chloride, polypropylene, polyester, or the like. The drug solution distribution tube 3 for continuous injection is provided with a connector to be connected to the drug solution port 2 of the reservoir 1 at one end and a connector to be connected to an epidural catheter or the like at the other end. The drug solution distribution tube 5 for additional administration is connected at one end to the drug solution distribution tube for continuous injection via a three-way connector or the like and is connected at the other end to the sub-reservoir 7 for storing drug solution to be additionally administered.

The sub-reservoir 7 has a press-deformed force and shape restoring force and is formed of flexible resin such as polyethylene, polypropylene, or polyester, or a rubbery resilient material such as polyisoprene, or silicone rubber or the like. The sub-reservoir 7 is connected to the other end of the drug solution distribution tube 5 for additional administration, and is also adapted to temporarily store drug solution discharged from the balloon 9 in the reservoir 1 . The shape may be a push-button type, which is easy for the patient to operate, or a bag formed of the above-described rubbery resilient material.

The flow rate control device 4 or 6 used in the present invention includes the release valve 21 or 31 which is opened by a certain pressure and allows drug solution to flow in and out therefrom, and the flow rate control tube 22 or 32 for adjusting the flow rate of drug solution flowing out from the reservoir by resistance in the duct line.

The release valve is a valve which is opened by a certain pressure and allows drug solution in the sub-reservoir, and is normally closed and is adapted to be opened when the internal pressure in the sub-reservoir exceeds a predetermined value. Release valves of this type include that of an umbrella type (JP-A-9-308687), a rubbery resilient film, or that of a duckbill type. However, when the flow rate control tube and the release valve as shown in Fig. 2 and Fig. 3 are provided coaxially, valves of the umbrella type (umbrella valve 21) or of the rubbery resilient film (rubbery resilient film 31) are preferable.

The umbrella valve 21 is high in tenacity and elasticity, and is formed preferably of silicone rubber or latex rubber, which is chemically resistant material and can hardly be damaged by the action of a drug solution. It is also preferable to set the pressure at which the valve is opened to a value relatively high so that the valves is not opened by the internal pressure of the balloon 9 in the reservoir 1, but at which the patient can easily discharged the drug solution as the maximum value.

The rubbery resilient film 31 is high in tenacity and elasticity, and is formed preferably of silicone rubber or latex rubber, which is a chemically resistant material and can hardly be damaged by the action of a drug solution. It is also preferable to set the pressure at which the film is opened to a value relatively high so that the films is not opened by the internal pressure of the balloon 9 in the reservoir 1, but at which the patient can easily discharge the drug solution at the maximum value.

The flow rate control tube 22 or 32, being a tube body of extremely small diameter, for adjusting the flow rate of drug solution to be flown out from the reservoir by resistance in the duct line, is generally formed of a synthetic resin such as a polyolefin, polyvinyl chloride, polyester, polyamide, polyurethane, polyacrylate, polycarbonate, or a metal. The size of the flow rate control tube is 10 to 500µ in inner diameter, and preferably 50 to 300µ. The length is preferably at least 1 cm, and the outer diameter is preferably 5 to 500 times the inner diameter. If the inner diameter of the flow rate control tube is smaller than 10µ, flow of the drug solution tends to stop because air in the drug solution is attached to the inner wall of the tube, while if the inner diameter exceeds 500µ, control of the flow rate of the drug solution tends to be difficult.

The check valve 8 is of a duckbill type in which the closed end is pointed as a bill of a water mole, and is adapted to allow drug solution extruded from the balloon 9 in the reservoir 1 to flow, but to prevent a reverse flow. In this case, drug solution stored in the sub-reservoir 7, which is forced out by the patient, is prevented from flowing in the reverse direction to the balloon 9, so that the drug solution of a controlled amount can be additionally administered. In addition to the valve of the duckbill type, an umbrella valve, a flap valve, poppet valve, ball valve and so on may be used, and materials which can be used for these valves are fluorine containing resin, nylon, polyolefin, polyvinyl chloride, polycarbonate, silicone resin, and so on.

The drug solution tube 3 for continuous injection extended from the drug solution port 2 includes two parts; a part (3a) to be connected to the drug solution port 2 of the reservoir 1; and a part (3b) to be connected to the flow rate control device 4, and the two parts are connected by the three-way connector 10 together with the drug solution distribution tube 5 for additional administration.

The PCA apparatus according to the present invention is not limited to the embodiments shown in the drawings as long as it has the above-described control devices 4 and 6 and can carry out continuous administration.

## Claims

1. A patient-controlled analgesia (PCA) apparatus capable of simultaneous continuous injection and additional administration of a drug solution, comprising:
(i) a reservoir (1) for storing the drug solution in a pressurized state and allowing the drug solution to flow into and out of a drug solution port (2);
(ii) a first drug solution distribution tube (3) for continuous injection extending from the drug solution port (2);
(iii) a first flow rate control device (4) including a first release valve (21; 31) disposed in the first drug solution distribution tube (3) for continuous injection and which is openable at a certain pressure for allowing the drug solution during additional administration to flow through and a first flow rate control tube (22; 32) for controlling a flow rate of the drug solution from the reservoir (1) through the first drug solution distribution tube (3) by resistance in the first flow rate control tube (22; 32) during continuous injection;
(iv) a second drug solution distribution tube (5) for additional administration branched from the first drug solution distribution tube (3) between the reservoir (1) and the first flow rate control device (4);
(v) a second flow rate control device (6) including a second flow rate control tube (22; 32) for controlling the flow rate of the drug solution from the reservoir (1) through the second drug solution distribution tube (5) to a sub-reservoir by resistance in the second flow rate control tube (22; 32); and
(vi) the sub-reservoir (7) capable of being pressed and deformed to discharge the drug solution provided at a distal end of the second drug solution distribution tube (5) for additional
wherein the first drug solution distribution (3) for continuous injection extending from the drug solution port (2) includes a portion (3a) to be connected to the drug solution port (2) of the reservoir (1) and a portion (3b) to be connected to the first flow rate control device (4), and is connected via a three-way connector (10) to the second drug solution distribution tube (5) for additional administration, **characterised in that** the second flow rate control device (6) includes a second release valve (21; 31) disposed in the second drug solution distribution tube (5) for additional administration which is openable at a certain pressure for allowing the drug solution during additional administration to flow through.

2. The patient-controlled analgesia (PCA) apparatus capable of simultaneous continuous injection and additional administration of drug solution according to Claim 1, further comprising a check valve (8) for preventing the drug solution from flowing to the reservoir (1) at the time of additional administration and provided in the first drug solution distribution tube (3) for continuous injection provided between the second drug solution distribution tube (5) for additional administration and the reservoir (1).

## Patentansprüche

1. Patienten-kontrollierte Analgesievorrichtung (PCA), welche zur gleichzeitigen kontinuierlichen Injektion und zur zusätzlichen Verabreichung einer Medikamentenlösung fähig ist, umfassend:
(i) einen Behälter (1) zum Speichern der Medikamentenlösung in einem unter Druck gesetzten Zustand und um es der Medikamentenlösung zu ermöglichen, aus einer Medikamentenlösungsöffnung (2) herein- und herauszuströmen,
(ii) eine erste Medikamentenlösungsverteilungsröhre (3) zur kontinuierlichen Injektion, welche sich von der Medikamentenlösungsöffnung (2) erstreckt,
(iii) eine erste Strömungsratensteuereinrichtung (4) umfassend ein erstes in der ersten Medikamentenlösungsverteilungsröhre (3) zur kontinuierlichen Injektion angeordnetes erstes Ablassventil (21; 31), welches bei einem bestimmten Druck geöffnet werden kann, um es der Medikamentenlösung während der zusätzlichen Verabreichung zu ermöglichen, hindurchzuströmen, und eine erste Strömungsratensteuerröhre (22; 32) zum Steuern einer Strömungsrate der Medikamentenlösung aus dem Behälter (1) durch die erste Medikamentenlösungsverteilungsröhre (3) aufgrund eines Widerstandes in der ersten Strömungsratensteuerröhre (22; 32) während der kontinuierlichen Injektion,
(iv) eine zweite Medikamentenlösungsverteilungsröhre (5) für die zusätzliche Verabreichung, welche von der ersten Medikamentenlösungsverteilungsröhre (3) zwischen dem Behälter (1) und der ersten Strömungsratensteuereinrichtung (4) abzweigt,
(v) eine zweite Strömungsratensteuereinrichtung (6) umfassend eine zweite Strömungsratensteuerröhre (22; 32) zum Steuern der Strömungsrate der Medikamentenlösung von dem Behälter (1) durch die zweite Medikamentenlösungsverteilungsröhre (5) zu einem Unterbehälter aufgrund von Widerstand in der zweiten Strömungsratensteuerröhre (22; 32), und
(vi) wobei der Unterbehälter (7) gedrückt und deformiert werden kann, um die bereitgestellte Medikamentenlösung an einem distalen Ende der zweiten Medikamentenlösungssteuerröhre (5) zur zusätzlichen Verabreichung abzugeben,
wobei die erste Medikamentenlösungsverteilungsröhre (3) zur kontinuierlichen Injektion, welche sich von der Medikamentenlösungsöffnung (2) erstreckt, einen Abschnitt (3a), welcher mit der Medikamentenlösungsöffnung (2) des Behälters (1) zu verbinden ist, und einen Abschnitt (3b), welcher mit der ersten Strömungsratensteuereinrichtung (4) zu verbinden ist, umfasst, und über einen Drei-Wege-Verbinder (10) mit der zweiten Medikamentenlösungsverteilungsröhre (5) zur zusätzlichen Verabreichung verbunden ist, **dadurch gekennzeichnet, dass** die zweite Strömungsratensteuereinrichtung (6) ein zweites Ablassventil (21; 31) umfasst, welches in der zweiten Medikamentenlösungsverteilungsröhre (5) zur zusätzlichen Verabreichung angeordnet ist und welches bei einem bestimmten Druck geöffnet werden kann, um es der Medikamentenlösung während der zusätzlichen Verabreichung zu ermöglichen, hindurchzuströmen.

2. Patienten-kontrollierte Analgesievorrichtung (PCA), welche zur gleichzeitigen kontinuierlichen Injektion und zur zusätzlichen Verabreichung einer Medikamentenlösung in der Lage ist, gemäß Anspruch 1, weiter umfassend ein Rückschlagventil (8), um zu verhindern, dass zu der Zeit der zusätzlichen Verabreichung die Medikamentenlösung zu dem Behälter (1) strömt, welches in der ersten Medikamentenlösungsverteilungsröhre (3) zur kontinuierlichen Verabreichung, welche zwischen der zweiten Medikamentenlösungsverteilungsröhre (5) zur zusätzlichen Verabreichung und dem Behälter (1) bereitgestellt ist, bereitgestellt ist.

## Revendications

1. Appareil d'analgésie contrôlée par le patient (PCA) pouvant réaliser simultanément une injection continue et une administration supplémentaire d'une solution médicamenteuse, comprenant :
(i) un réservoir (1) pour stocker la solution médicamenteuse dans un état mis sous pression et permettant à la solution médicamenteuse de s'écouler à l'intérieur et à l'extérieur d'un orifice (2) de solution médicamenteuse ;
(ii) un premier tube de distribution de solution médicamenteuse (3) pour l'injection continue, s'étendant à partir de l'orifice (2) de solution médicamenteuse ;
(iii) un premier dispositif de régulation de débit (4) comprenant une première soupape de sûreté (21 ; 31) disposée dans le premier tube de distribution de solution médicamenteuse (3) pour l'injection continue et qui peut s'ouvrir à une certaine pression pour permettre à la solution médicamenteuse, pendant l'administration supplémentaire de s'écouler à travers celle-ci et un premier tube de régulation de débit (22 ; 32) pour réguler un débit de la solution médicamenteuse à partir du réservoir (1) en passant par le premier tube de distribution de solution médicamenteuse (3) par la résistance dans le premier tube de régulation de débit (22 ; 32) pendant l'injection continue ;
(iv) un second tube de distribution de solution médicamenteuse (5) pour l'administration supplémentaire dérivée du premier tube de distribution de solution médicamenteuse (3) entre le réservoir (1) et le premier dispositif de régulation de débit (4) ;
(v) un second dispositif de régulation de débit (6) comprenant un second tube de régulation de débit (22 ; 32) pour réguler le débit de la solution médicamenteuse à partir du réservoir (1) en passant par le second tube de distribution de solution médicamenteuse (5) jusqu'à un sous-réservoir par la résistance dans le second tube de régulation de débit (22 ; 32) ; et
(vi) le sous-réservoir (7) peut être comprimé et déformé pour décharger la solution médicamenteuse prévue à une extrémité distale du second tube de distribution de solution médicamenteuse (5) pour l'administration supplémentaire,
dans lequel le premier tube de distribution de solution médicamenteuse (3) pour l'injection continue, s'étendant à partir de l'orifice (2) de solution médicamenteuse comprend une partie (3a) à raccorder à l'orifice (2) de solution médicamenteuse du réservoir (1) et une partie (3b) à raccorder au premier dispositif de régulation de débit (4), et est raccordé via un connecteur à trois voies (10) à un second tube de distribution de solution médicamenteuse (5) pour l'administration supplémentaire, **caractérisé en ce que** le second dispositif de régulation de débit (6) comprend une seconde soupape de sûreté (21 ; 31) disposée dans le second tube de distribution de solution médicamenteuse (5) pour l'administration supplémentaire qui peut s'ouvrir à une certaine pression pour permettre à la solution médicamenteuse, pendant l'administration supplémentaire, de s'écouler à travers celle-ci.

2. Appareil d'analgésie contrôlée par le patient (PCA) pouvant réaliser simultanément une injection continue et une administration supplémentaire d'une solution médicamenteuse selon la revendication 1, comprenant en outre une soupape anti-retour (8) pour empêcher la solution médicamenteuse de s'écouler dans le réservoir (1) au moment de l'administration supplémentaire et prévue dans le premier tube de distribution de solution médicamenteuse (3) pour l'injection continue prévu entre le second tube de distribution de solution médicamenteuse (5) pour l'administration supplémentaire et le réservoir (1).
